(19) Europäisches Patentamt
European Patent Office
Office européen des brevets

(11) EP 0 416 365 B1

(12) **EUROPÄISCHE PATENTSCHRIFT**

(45) Veröffentlichungstag und Bekanntmachung des Hinweises auf die Patenterteilung: **31.01.1996 Patentblatt 1996/05**

(51) Int. Cl.$^6$: **C07C 233/60**, A01N 37/18

(21) Anmeldenummer: **90115970.7**

(22) Anmeldetag: **21.08.1990**

(54) **Derivate carbocyclischer Anilide**
Carbocyclic anilide derivatives
Dérivés d'anilide carbocyclique

(84) Benannte Vertragsstaaten:
**BE CH DE DK FR GB IT LI NL**

(30) Priorität: **02.09.1989 DE 3929231**
**21.04.1990 DE 4012712**

(43) Veröffentlichungstag der Anmeldung:
**13.03.1991 Patentblatt 1991/11**

(73) Patentinhaber: **BAYER AG**
**D-51368 Leverkusen (DE)**

(72) Erfinder:
- **Krüger, Bernd-Wieland, Dr.**
  **D-5060 Bergisch-Gladbach 2 (DE)**
- **Sasse, Klaus, Dr.**
  **D-5060 Bergisch-Gladbach 2 (DE)**
- **Brandes, Wilhelm, Dr.**
  **D-5653 Leichlingen 1 (DE)**

(56) Entgegenhaltungen:
**EP-A- 0 100 615** **EP-A- 0 125 901**
**EP-A- 0 293 718** **DE-A- 2 018 783**
**FR-A- 1 588 718**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 416 365 B1

## Beschreibung

Die vorliegende Erfindung betrifft neue Cycloalkyl bzw. Cycloalkenylcarbonsäureanilide, ein Verfahren zu ihrer Herstellung und ihre Verwendung zur Bekämpfung von Schädlingen, insbesondere von Pilzen.

Es ist bekannt, daß bestimmte Carbamate gute fungizide Eigenschaften besitzen (vgl. EP 293 718, FR-1 588718).

Weiterhin sind viele Carbonsäureanilide mit fungizider Wirkung, insbesondere mit hoher Wirkung gegen Benzimidazol-tolerante Pflanzenpathogene, bekannt (vgl. EP 117 024, EP 125 901, EP 100 615).

Es wurden neue Cycloalkyl- bzw. Cycloalkenyl-carbonsäureanilide der allgemeinen Formel (I)

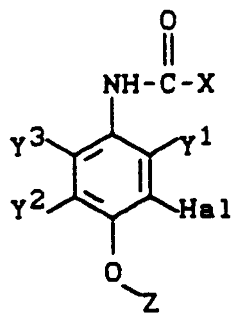

(I)

in welcher

| | |
|---|---|
| X | für gegebenenfalls Alkyl-substituiertes Cyclopentyl, Cyclohexyl oder Cycloheptyl oder gegebenenfalls Alkyl-substituiertes Cycloalkenyl steht, |
| Hal | für Halogen steht und |
| $Y^1$, $Y^2$ und $Y^3$ | unabhängig voneinander für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen und |
| Z | für die Gruppen $COOR^2$ oder $COR^1$ steht, wobei |
| $R^1$ und $R^2$ | gleich oder verschieden sind und für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl stehen |

gefunden.

Die substituierten Cycloalkyl- bzw. Cycloalkenyl-carbonsäureanilide der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen substituierten Cycloalkyl- bzw. Cycloalkenylcarbonsäureanilide der Formel (I)

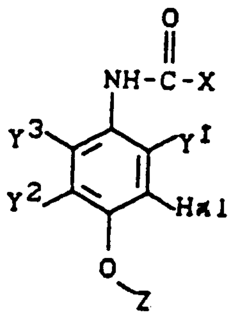

(I)

in welcher

Z, X, $Y^1$, $Y^2$, $Y^3$ und Hal die oben angegebene Bedeutung haben

erhält, wenn man Aminophenole der Formel (II)

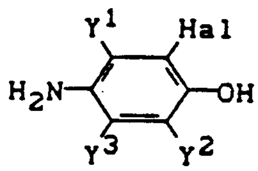

(II)

in welcher

Hal, $Y^1$, $Y^2$ und $Y^3$ die oben angegebenen Bedeutungen haben, in einem ersten Reaktionsschritt

mit Carbonsäure-Derivaten der Formel (III)

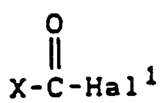

(III)

in welcher

X die oben angegebene Bedeutung hat und

$Hal^1$ für Halogen, vorzugsweise Chlor, oder eine bei Acylierungsreaktionen gebräuchliche Abgangsgruppe, vorzugsweise einen aktivierenden Esterrest, steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt diese erhaltenen Zwischenprodukte der Formel (IV)

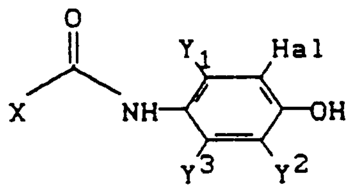

(IV)

in welcher

X, $Y^1$, $Y^2$, $Y^3$ und Hal die obengenannte Bedeutung haben,

mit Carbonyl-Derivaten der Formel (V)

$$Z\text{-}Hal^2 \qquad (V)$$

in welcher

Z die obengenannte Bedeutung hat und

$Hal^2$ für Halogen, vorzugsweise Chlor, oder eine bei Acylierungsreaktionen gebräuchliche Abgangsgruppe, vorzugsweise einen aktivierenden Esterrest, steht,

gegebenenfalls in Gegenwart eines Säureakzeptors und gegebenenfalls in Gegenwart eines Lösungs- oder Verdünnungsmittels umsetzt.

Schließlich wurde gefunden, daß die substituierten Cycloalkyl- bzw. Cycloalkenyl-carbonsäureanilide der Formel (I) und (IVa) u. a. eine hohe fungizide Wirksamkeit besitzen. Die neuen Verbindungen können auch mit anderen bekannten, hochwirksamen Verbindungen in synergistischen Mischungen verwendet werden.

Im Rahmen der vorliegenden Erfindung besitzen die Substituenten vorzugsweise die folgenden Bedeutungen:

Halogen kann, überall wo nicht anders angegeben, Fluor, Chlor, Brom und Iod, bevorzugt Fluor, Chlor und Brom bedeuten.

Alkyl, Alkoxy und Alkylthio stehen für einen Rest, der je Alkyleinheit 1 - 8, bevorzugt 1 - 6 und besonders bevorzugt 1 - 4 Kohlenstoffatome hat, z. B. Methyl, Ethyl, n- und iso-Propyl, n-, sec-, iso- und tert.-Butyl, Pentyl, n-Hexyl oder iso-Hexyl, Methoxy, Ethoxy, n- und iso-Propoxy, n-, sec-, iso- und tert.-Butoxy, Pentoxy und Hexoxy, Methylthio, Ethylthio, n- und iso-Propylthio, n-, sec.-, iso- und tert.-Butylthio, Pentylthio und Hexylthio.

Halogenalkoxy bzw. Halogenalkylthio steht im allgemeinen für einen über Sauerstoff bzw. Schwefel gebundenen, geradkettigen oder verzweigten Kohlenwasserstoffrest mit 1 - 6 Kohlenstoffatomen und 1 - 9 gleichen oder verschiedenen Halogenatomen. Bevorzugt sind Reste mit 1 - 4 Kohlenstoffatomen und 1 - 5 gleichen oder verschiedenen Halogenatomen. Ganz besonders bevorzugt sind Reste mit 1 oder 2 Kohlenstoffatomen und 1 - 3 gleichen oder verschiedenen Halogenatomen. Beispielhaft seien genannt: Trifluormethoxy, Trichlormethoxy, Difluorchlormethoxy, Dichlorfluormethoxy, Difluorethoxy, Trifluorethoxy, Tetrafluorethoxy, Pentafluorethoxy, Trifluormethylthio, Trichlormethylthio, Difluorchlormethylthio, Dichlorfluormethylthio, Difluorethylthio, Trifluormethylthio, Tetrafluorethylthio.

Halogenalkyl hat die Bedeutung von Halogenalkoxy, mit dem Unterschied, daß das Sauerstoff bzw. Schwefelatom fehlt.

Cycloalkyl steht im allgemeinen für einen cyclischen Kohlenwasserstoffrest mit 3 - 10 Kohlenstoffatomen. Bevorzugt sind Reste mit 3 - 7 Kohlenstoffatomen. Beispielhaft seien genannt: Cyclopropyl, Cyclobutyl, Cyclopentyl, Cyclohexyl, Cycloheptyl und Cyclodecanyl.

Die Cycloalkylreste können ein- bis mehrfach substituiert sein. Als Substituenten seien Alkyl mit 1 - 4 Kohlenstoffatomen Halogen, Halogenalkyl mit 1 bis 4 Kohlenstoffatomen und 1 bis 9 gleichen oder verschiedenen Halogenatomen, Alkoxy mit 1 bis 4 Kohlenstoffatomen, Hydroxy und Alkylcarbonylox mit 1 bis 6 Kohlenstoffatomen im Alkylteil genannt.

Cycloalkenyl steht im allgemeinen für einen cyclischen Kohlenwasserstoffrest mit 5-10 Kohlenstoffatomen.

Bevorzugt sind Reste mit 5-7 Kohlenstoffatomen. Beispielhaft seien genannt: Cyclopentenyl, Cyclohexenyl und Cycloheptenyl.

Die Cycloalkenylreste können ein- bis mehrfach substituiert sein. Als Substituenten seien Alkyl mit 1-6 Kohlenstoffatomen genannt.

Alkyl hat hierbei die bevorzugte und besonders bevorzugte Bedeutung, die bereits weiter oben gegeben wurde.

Phenyl Phenylalkyl und substituiertes Phenoxyalkyl stehen im allgemeinen für Phenyl, Phenylalkyl und Phenoxyalkyl, in denen Phenylwasserstoffatome gegebenenfalls durch einen oder mehrere Substituenten $Y^{1'}$-$Y^{5'}$ ersetzt sind. $Y^{1'}$-$Y^{5'}$ haben hierbei die Bedeutung von $Y^1$, $Y^2$ und $Y^3$ sowie Nitro und Cyano.

Alkenyl steht im allgemeinen für einen geradkettigen oder verzweigten Kohlenwasserstoffrest mit 2 bis 8 Kohlenstoffatomen und einer oder mehreren, bevorzugt mit einer oder zwei, Doppelbindungen. Bevorzugt ist Niederalkenyl mit 2 bis 6 Kohlenstoffatomen und einer Doppelbindung. Besonders bevorzugt ist ein Alkenylrest mit 2 bis 5 Kohlenstoffatomen und einer Doppelbindung.

Die erfindungsgemäßen substituierten Cycloalkyl- bzw. Cycloalkenyl-carbonsäureanilide sind durch die Formel (I) allgemein definiert. Bevorzugt sind Verbindungen der Formel (I), worin

X für gegebenenfalls ein- bis vierfach, gleich oder verschieden durch geradkettiges oder verzweigtes Alkyl mit 1 - 4 Kohlenstoffatomen substituiertes Cyclopentyl, Cyclohexyl oder Cycloheptyl steht oder für Cycloalkenyl mit 5 - 7 Ringgliedern steht, wobei der Cycloalkylrest bzw. Cycloalkenylrest ein- bis sechsfach , gleich oder verschieden durch geradkettiges oder verzweigtes Alkyl mit 1 - 4 Kohlenstoffatomen substituiert sein kann,

Hal für Fluor, Chlor oder Brom steht,

$Y^1$, $Y^2$ und $Y^3$ gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 - 4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen,

Z für $COOR^2$ oder $COR^1$ steht, wobei

$R^1$ und $R^2$ gleich oder verschieden sind und für gegebenenfalls einfach bis neunfach durch Halogen substituiertes $C_1$-$C_8$-Alkyl, $C_2$-$C_8$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl stehen oder für unsubstituiertes oder einfach bis fünffach, gleich oder verschieden durch $C_1$-$C_4$-Halogenalkyl, Halogen, $C_1$-$C_4$-Alkyl, $C_1$-$C_4$-Alkoxy, Hydroxy, $C_1$-$C_6$-Alkylcarbonyloxy substituiertes $C_3$-$C_7$-Cycloalkyl stehen oder für unsubstituiertes oder im Phenylteil einfach bis fünffach, gleich oder verschieden durch $Y^{1'}$-$Y^{5'}$ substituiertes Phenyl-$C_1$-$C_4$-alkyl oder für unsubstituiertes oder im Phenylteil einfach bis fünffach, gleich oder verschieden durch $Y^{1'}$-$Y^{5'}$ substituiertes Phenoxyalkyl stehen,
wobei $Y^{1'}$-$Y^{5'}$ die Bedeutung von $Y^1$-$Y^3$, $NO_2$ und Cyano haben.

Besonders bevorzugt sind Verbindungen der Formel (I), worin

X für ein- oder zweifach, gleich oder verschieden durch geradkettiges oder verzweigtes Alkyl mit 1 - 4 Kohlenstoffatomen substituiertes Cyclopentyl, Cyclohexyl oder Cycloheptyl, für gegebenenfalls ein- oder zweifach, gleich oder verschieden durch geradkettiges oder verzweigtes Alkyl mit 1 - 4 Kohlenstoffatomen substituiertes Cyclopentenyl, Cyclohexenyl, Cycloheptenyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht,

Z für $COOR^2$ oder $COR^1$ steht, wobei
$R^1$ und $R^2$ gleich oder verschieden sind und für $C_1$-$C_6$-Alkyl, $C_2$-$C_6$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_2$-alkyl stehen oder für unsubstituiertes oder einfach bis dreifach, gleich oder verschieden durch Fluor, Chlor, Brom, $C_1$-$C_2$-Alkyl, $C_1$-$C_2$-Alkoxy, $C_1$-$C_6$-Alkylcarbonyloxy substituiertes $C_3$-$C_7$-Cycloalkyl stehen oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl-$C_1$-$C_2$-alkyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenoxy-$C_1$-$C_2$-alkyl stehen.

Ganz besonders bevorzugt sind Verbindungen der Formel (I), worin

X für in 1- oder 1,3-Stellung durch Methyl oder Ethyl substituiertes Cyclopentyl, Cyclohexyl, Cycloheptyl, Cyclopentenyl, Cyclohexenyl, Cycloheptenyl steht, welche gegebenenfalls durch einen weiteren Alkylrest mit 1 - 3 Kohlenstoffatomen zusätzlich substituiert sind,

Hal für Fluor, Chlor oder Brom steht und

$Y^1$, $Y^2$ und $Y^3$ gleich oder verschieden sind und für Wasserstoff, Fluor, Chlor, Brom oder Trifluormethyl stehen,

Z für $COOR^2$ oder $COR^1$ steht, wobei
$R^1$ und $R^2$ gleich oder verschieden sind und für $C_1$-$C_4$-Alkyl, $C_2$-$C_5$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl stehen oder für einfach bis dreifach, gleich oder verschieden durch Methoxy, Fluor, Chlor, Brom, Methyl, $C_1$-$C_4$-Alkylcarbonyloxy substituiertes $C_3$-$C_7$-Cycloalkyl stehen oder für einfach bis dreifach, gleich oder verschieden durch Nitro, Cyano, Fluor, Chlor, Brom, Methyl, Methoxy substituiertes Phenyl oder für einfach bis dreifach, gleich oder verschieden durch Nitro, Cyano, Fluor, Chlor, Brom, Methyl, Methoxy substituiertes Phenylmethyl stehen.

Verwendet man beispielsweise 2,6-Dichlor-4-amino-phenol, 1-Methyl-1-chlorcarbonylcyclohexan und Chlorameisensäurebutylester als Ausgangsstoffe, so kann der Reaktionsverlauf durch das folgende Formelschema wiedergegeben werden:

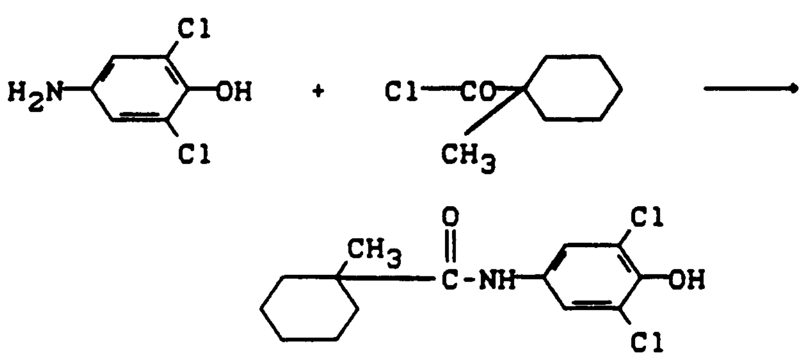

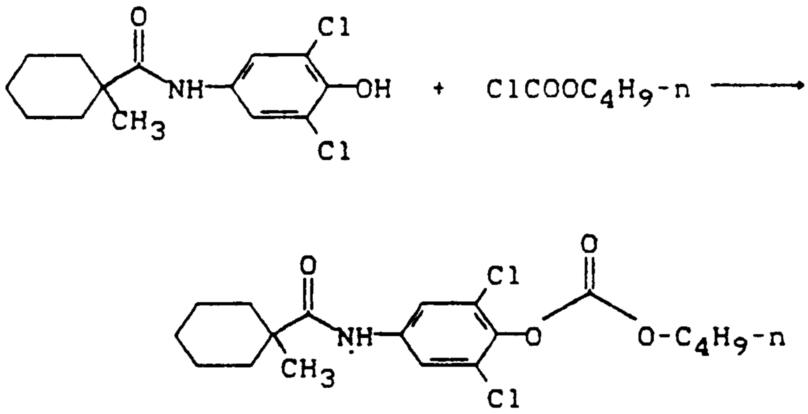


Die zur Durchführung des erfindungsgemäßen Verfahrens als Ausgangsstoffe benötigten Aminophenole sind durch die Formel (II) allgemein definiert. In dieser Formel (II) haben die Reste Hal und $Y^1$ - $Y^3$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar (vergl. "Methoden der organischen Chemie", Houben-Weyl, Band VI/1c, Phenole, Teil 1, Georg Thieme Verlag, Stuttgart, 1976, und "Reaktionen der organischen Synthese", Cesare Ferri, S. 81, 89, 91, 97, 118, 120, 122, 124, 126, 128, Georg Thieme Verlag, Stuttgart, 1978).

Die 4-Amino-2-chlor- bzw. -2-brom-6-trifluormethylphenole sind bekannt aus Jp. Kokai Tokkyo Koho Jp 61/126055 und z. B. 4-Amino-2,3,5,6-tetrafluorphenol aus Zh. Org. Khim. 10(9), 1923-1927 (1974). Die Verbindungen der Formel (II A)

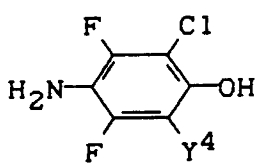

(II A)

in welcher

$Y^4$ für Fluor oder Chlor steht, sind Gegenstand von EP-A-293 718 und werden z.B. hergestellt aus entsprechenden Hydroxybenzoesäuren der Formel (VA)

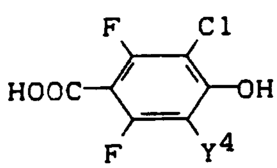

(VA)

durch Decarboxylierung mit anschließender Nitrierung der entstehenden Phenole der Formel (VI A)

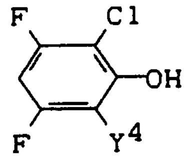

(VI A)

zu den Nitroverbindungen der Formel (VII A)

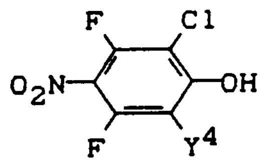

(VII A)

die dann hydriert werden, z. B. mit Wasserstoff und Raney-Nickel, zu den entsprechenden Aminen der Formel (II A).

Auch die Verbindungen der Formel (VII A) sind Gegenstand der EP-A-293 718 europäischen Anmeldung.

Die zur Durchführung des erfindungsgemäßen Verfahrens außerdem benötigten Cycloalkancarbonsäure- bzw. Cycloalkencarbonsäure-Derivate sind durch die Formel (III), in der X für Cycloalkyl oder Cycloalkenyl steht, allgemein definiert. In dieser Formel (III) haben die Reste X und $Hal^1$ die bereits im Zusammenhang mit der Beschreibung der erfindungsgemäßen Verbindungen der Formel (I) gegebenen Bedeutungen. Die Verbindungen sind bekannt und nach Analogieverfahren herstellbar (vergl. Diversi et. al., Synthesis 1971, 258; US 3 674 831; "Reaktionen der organischen Synthese" Cesare Ferri, S. 460, 461, 1978, Georg Thieme Verlag, Stuttgart); Houben-Weyl, Methoden der organischen Chemie, Bd. E5 Tl.1, S.211, 320, 343, 428 ff, G. Thieme-Verlag, Stuttgart, 1985).

Die zur Durchführung des erfindungsgemäßen Verfahrens außerdem benötigten Carbonsäure- und Kohlensäure-Derivate der Formel (V), in welcher Z und $Hal^2$ die oben angegebene Bedeutung haben sind bekannt und nach Analogieverfahren herstellbar (vgl. "Reaktionen der organischen Synthese" Cesare Ferri, S. 432 ff, 460 ff 1978, Georg Thieme Verlag, Stuttgart; Houben-Weyl, "Methoden der organischen Chemie, Kohlensäure-Derivate, Bd. E4, S. 9 ff, Georg Thieme Verlag, Stuttgart, 1983; Bd. E5 Tl. 1, S. 193 ff, Georg Thieme Verlag, Stuttgart, 1985).

Die bei dem erfindungsgemäßen Verfahren als Zwischenprodukte verwendeten Acylaminophenole sind durch die Formel (IV) definiert.

Unter den Verbindungen der Formel (IV) sind die Verbindungen der Formel (IVa)

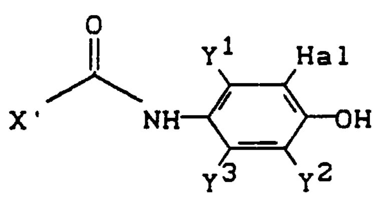

(IVa)

in denen

| | |
|---|---|
| X′ | für einen gegebenenfalls Alkyl-substituierten Cycloalkenylrest, vorzugsweise einen $C_1$-$C_2$-Alkyl-substituierten Cyclopentenyl-, Cyclohexenyl- oder Cycloheptenylrest, steht, und |
| $Y^1$, $Y^2$, $Y^3$ und Hal | die obengenannte Bedeutung haben, neu. |

Man erhält die neuen Acylamino-Derivate der Formel (IVa), indem man Aminophenole der Formel (II)

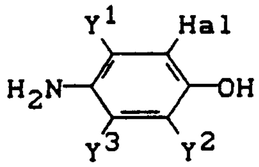

(II)

in welcher

$Y^1$, $Y^2$, $Y^3$ und Hal die obengenannte Bedeutung haben, mit Carbonsäuren-Derivaten der Formel (IIIa)

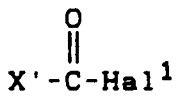

(IIIa)

in welcher

X′ und $Hal^1$ die obengenannte Bedeutung haben,

gegebenenfalls in Gegenwart eines Lösungsmittels und gegebenenfalls in Gegenwart eines Säureakzeptors umsetzt.

Die übrigen Verbindungen der Formel (IV) können entsprechend oder nach bekannten Verfahren erhalten werden.

Das erfindungsgemäße Verfahren wird gegebenenfalls in Gegenwart von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische aromatische und heterocyclische Amine, beispielsweise Triethylamin, Trimethylamin, Dimethylanilin, 1,8-Diazabicyclo-(5,4,0)-undec-7-en, Dimethylbenzylamin und Pyridin.

Zur Durchführung des erfindungsgemäßen Verfahrens setzt man im ersten Reaktionsschritt vorzugsweise auf ein Mol Amino-Phenol der allgemeinen Formel (II) 1 - 2 Mol, insbesondere 1 - 1,4 Mol, der Verbindungen der allgemeinen Formel (III) bzw. (IIIa) ein.

Für den zweiten Reaktionsschritt des erfindungsgemäßen Verfahrens setzt man vorzugsweise auf 1 Mol Acylaminophenol der Formel (IV) bzw. (IVa) 1-2 Mol, insbesondere 1-1,4 Mol, der Verbindungen der allgemeinen Formel (V) ein.

Als Verdünnungsmittel zur Durchführung des erfindungsgemäßen Verfahrens kommen praktisch alle inerten organischen Verdünnungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und - ethylester, Nitrile wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren wird im allgemeinen bei Temperaturen zwischen -50°C und 120°C durchgeführt. Bevorzugt wird der Bereich zwischen 0° C und 110° C. Die Umsetzungen werden im allgemeinen bei Normaldruck durchgeführt.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillieren", d. h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z. B. durch Kristallisation. Zur Charakterisierung der Verbindungen dienen Brechungsindex, Schmelzpunkt, $R_f$-Wert oder Siedepunkt.

Die erfindungsgemäßen Wirkstoffe sind für den Gebrauch zur Bekämpfung von Schädlingen, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Botrytispilzen an Bohnen sowie zur Bekämpfung von Getreidekrankheiten, wie beispielsweise gegen Pseudocercosporella einsetzen.

Die Wirkstoffe eignen sich weiterhin zur Bekämpfung von tierischen Schadlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwickıungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel 1

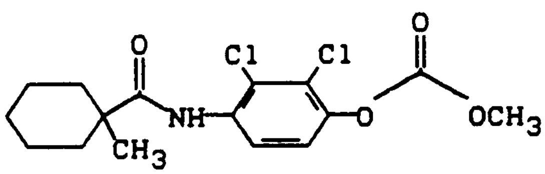

4 g (13,0 mMol) 2,3-Dichlor-4-(1-methyl-cyclohexyl-carbonyl)-amino-phenol werden in 50 ml Tetrahydrofuran gelöst und mit 2,5 ml (18,5 mMol) Triethylamin versetzt. Anschließend werden bei 20°C 1,5 g (14,3 mMol) Chlorameisensäuremethylester zur Reaktionsmischung getropft. Man rührt zwei Stunden bei 20°C und destilliert dann das Lösungsmittel im Wasserstrahlvakuum ab. Der ver bleibende Rückstand wird dann über Kieselgel chromatographiert (Laufmittel: Petrolether: Aceton = 8:2). Ausbeute: 4,4 g (98 % d. Th.). Fp.: 125°C.

Analog werden die folgenden Verbindungen der Formel (I)

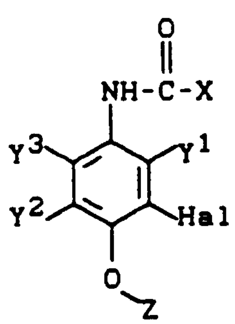

(I)

hergestellt:

| Beispiel Nr. | X | $Y^1$ | $Y^2$ | $Y^3$ | Hal | Z | phys. Daten (Fp.) |
|---|---|---|---|---|---|---|---|
| 2 | 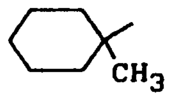 | Cl | H | H | Cl | $COOCH_2CH(CH_3)_2$ | 109° C |
| 3 | 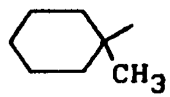 | Cl | H | H | Cl | $COOC_3H_7n$ | 122° C |
| 4 | 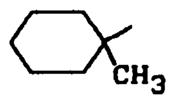 | Cl | H | H | Cl | $COOCH_2CH(CH_3)C_2H_5$ | 67° C |
| 5 | 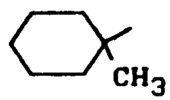 | Cl | H | H | Cl | 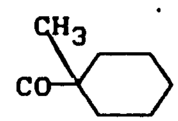 | 63° C |
| 6 | 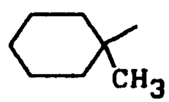 | Cl | H | H | Cl | 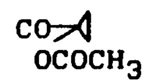 | 91° C |
| 7 | 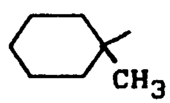 | Cl | H | H | Cl | 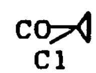 | 107° C |

| Beispiel Nr. | X | $Y^1$ | $Y^2$ | $Y^3$ | Hal | Z | phys. Daten (Fp.) |
|---|---|---|---|---|---|---|---|
| 8 | 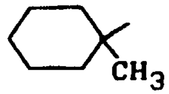 | Cl | H | H | Cl | 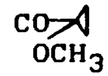 | 125° C |
| 9 | 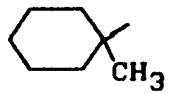 | Cl | H | H | Cl | 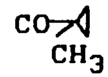 | 110° C |
| 10 | 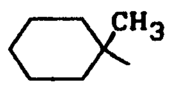 | Cl | H | H | Cl | $COCH_3$ | 105° C |
| 11 | 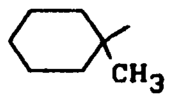 | Cl | H | H | Cl | $COC_4H_9$-t | 136° C |
| 12 | 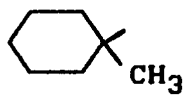 | Cl | H | H | Cl | $COCH(C_2H_5)C_4H_9$-n | 45° C |
| 13 | 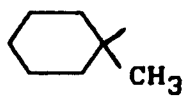 | Cl | H | H | Cl | 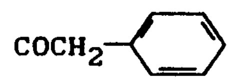 | 71° C |

| Beispiel Nr. | X | $Y^1$ | $Y^2$ | $Y^3$ | Hal | Z | phys. Daten (Fp.) |
|---|---|---|---|---|---|---|---|
| 14 | 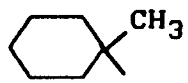 | Cl | H | H | Cl | 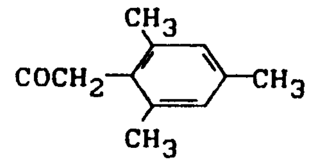 | 102° C |
| 15 | 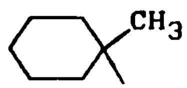 | Cl | H | H | Cl | 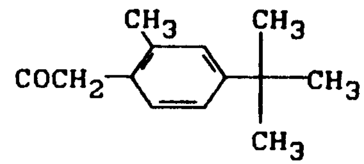 | 110° C |
| 16 | 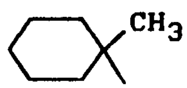 | Cl | H | H | Cl | 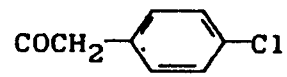 | 108° C |
| 17 | 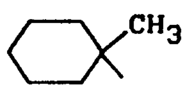 | Cl | H | H | Cl | 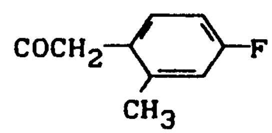 | 66° C |
| 18 | 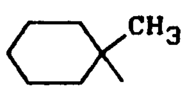 | Cl | H | H | Cl | 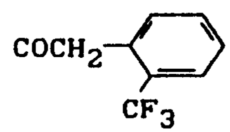 | 90° C |

| Beispiel Nr. | X | $Y^1$ | $Y^2$ | $Y^3$ | Hal | Z | phys. Daten (Fp.) |
|---|---|---|---|---|---|---|---|
| 19 | 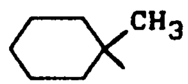 | Cl | H | H | Cl | 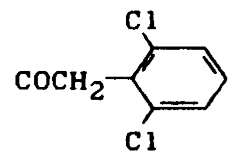 | 145° C |
| 20 | 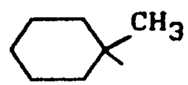 | Cl | H | H | Cl | 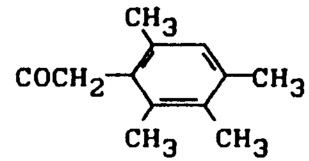 | 104° C |
| 21 | 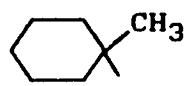 | Cl | H | H | Cl | 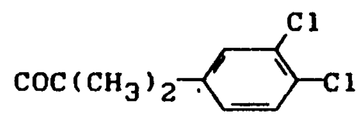 | 82° C |
| 22 | 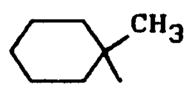 | Cl | H | H | Cl | 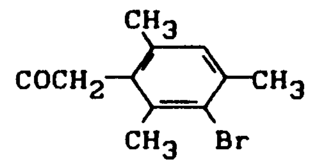 | 128° C |
| 23 | 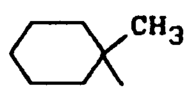 | Cl | H | H | Cl | 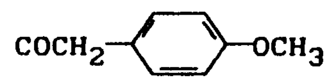 | 122° C |

| Beispiel Nr. | X | $Y^1$ | $Y^2$ | $Y^3$ | Hal | Z | phys. Daten (Fp.) |
|---|---|---|---|---|---|---|---|
| 24 | 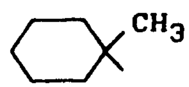 | Cl | H | H | Cl | 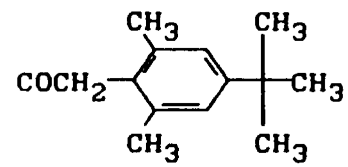 | 93° C |
| 25 | 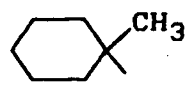 | F | F | F | Cl | 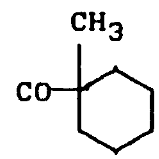 | 35° C |
| 26 | 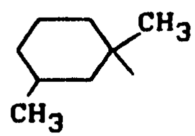 | Cl | H | H | Cl | $COOCH_3$ | |
| 27 | 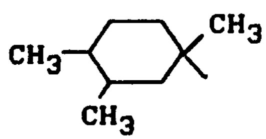 | Cl | H | H | Cl | $COOCH_3$ | |
| 28 | 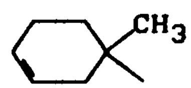 | Cl | H | H | Cl | $COOCH_3$ | |

| Beispiel Nr. | X | $Y^1$ | $Y^2$ | $Y^3$ | Hal | Z | phys. Daten (Fp.) |
|---|---|---|---|---|---|---|---|
| 29 | 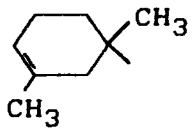 | Cl | H | H | Cl | $COOCH_3$ | |
| 30 | 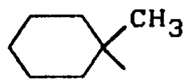 | Cl | H | H | Cl | COO 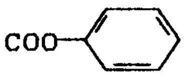 | |
| 31 | 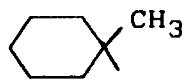 | Cl | H | H | Cl | $COOCH_2$ 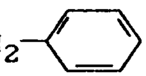 | |
| 32 | 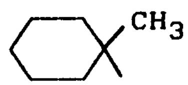 | Cl | H | H | Cl | $COCH_2O$ 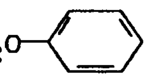 | |
| 33 | 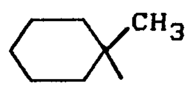 | Cl | H | H | Cl | $COCH_2$ 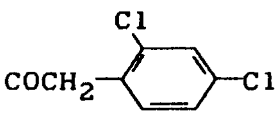 | 72° C |
| 34 | 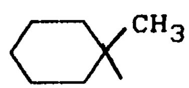 | H | Cl | H | Cl | $COOCH_2CH_2OC_2H_5$ | |
| 35 | 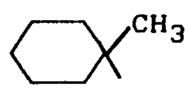 | Cl | H | H | Cl | $COOCH_2CH_2OC_2H_5$ | 52° C |

| Beispiel Nr. | X | $Y^1$ | $Y^2$ | $Y^3$ | Hal | Z | phys. Daten (Fp.) |
|---|---|---|---|---|---|---|---|
| 36 | 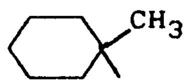 | Cl | H | H | Cl | $COOCH_2CH_2OCH_3$ | |
| 37 | 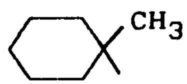 | Cl | H | H | Cl | $COOCH_2CH_2OC_3H_7$-i | |
| 38 | 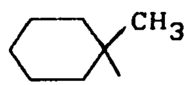 | Cl | H | H | Cl | $COOCH_2CH_2OC_4H_9$-n | |
| 39 | 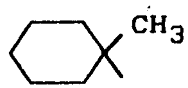 | Cl | H | H | Cl | $COOCH_2CH_2OCH(CH_3)C_2H_5$ | |
| 40 | 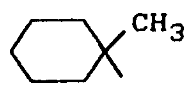 | Cl | H | H | Cl | $COOCH_2CH_2OC_3H_7$-n | |
| 41 | 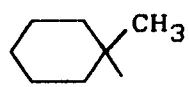 | Cl | H | H | Cl | $COOCH_2CH(CH_3)OCH_3$ | |
| 42 | 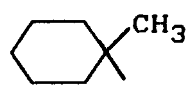 | Cl | H | H | Cl | $COOCH_2CH_2OCH_2CH_2OC_2H_5$ | $n_D^{20}$ 1,5116 |
| 43 | 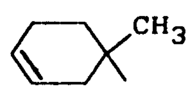 | Cl | H | H | Cl | $COOCH_2CH_2OC_2H_5$ | |

Herstellungsbeispiele

Herstellung der Ausgangsverbindungen:

Beispiel A1:

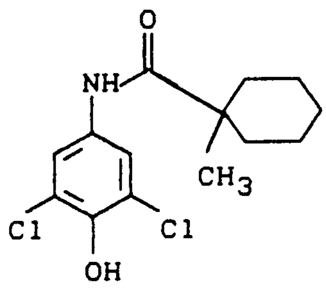

18,5 g (0,085 Mol) 4-Amino-2,6-dichlorphenol werden in 150 ml Tetrahydrofuran gelöst und zunächst mit 8,6 g (0,085 Mol) Triethylamin, dann bei 0°C Innentemperatur mit 15 g (0,094 Mol) 1-Methyl-cyclohexancarbonsäurechlorid versetzt. Man rührt über Nacht bei 20°C und fügt dann zur Vervollständigung der Reaktion erneut 5 g Carbonsäurechlorid und 2,8 g Triethylamin zur Reaktionsmischung. Nach 2 Stunden wird auf Eis gegossen und der abgesaugte Feststoff aus Toluol umkristallisiert. Man erhält die obengenannte Verbindung mit dem Schmelzpunkt 140°C; Ausbeute: 22,3 g (= 87 % der Theorie).

Analog werden die Verbindungen der Formel (IV) bzw. (IVa) erhalten:

Beispiel A2

3,5-Dichlor-2,6-difluor-4-hydroxybenzoesäure

In einer Rührapparatur werden 300 g Kaliumhydroxid, 600 ml Wasser, 15 g Tetrabutylammoniumchlorid und 135 g 3,5-Dichlor-2,4,6-trifluorbenzotrifluorid vorgelegt und dann für 5 Stunden am Rückfluß erhitzt. Nach Ende der Reaktion wird abgekühlt und durch Zutropfen von Salzsäure sauer gestellt. Das Festprodukt wird abgesaugt und im Vakuum getrocknet. Ausbeute: 93 g mit einem Schmelzpunkt 102 - 105°C.

Beispiel A3

3-chlor-2,5,6-trifluor-4-hydroxy-benzoesäure

Analog Beispiel A1 werden aus 400 g NaOH, 1200 ml Wasser, 15 g Tetraethylammoniumchlorid und 276 g 3-Chlortetrafluorbenzotrifluorid bei 6-stündigem Erhitzen unter Rückfluß 238 g Produkt erhalten mit einem Schmelzpunkt von 87 - 90°C.

Beispiel A4

2,6-Dichlor-3,5-difluorphenol

50 g 3,5-Dichlor-2,6-difluor-4-hydroxy-benzoesäure und 10 ml Dimethylformamid werden vermischt und erhitzt. Bei 105 - 130°C entwickelt sich Kohlendioxid und man läßt bei dieser Temperatur ausreagieren. Anschließend rührt man 200 ml Toluol und danach 80 ml Wasser ein, trennt die Phasen, trocknet die organische Phase und destilliert anschließend. Man erhält 34 g des Produktes mit einem Siedepunkt von 87 - 88°C und einem Brechungsindex von $n_D^{20}$ : 1,5310.

Beispiel A5

Analog Beispiel A3 erhält man 2-Chlor-3,5,6-trifluorphenol, mit einem Siedepunkt 68 - 70°C / 20 mbar.

Beispiel A6

2,6-Dichlor-3,5-difluor-4-nitro-phenol

In 70 ml Essigsäure werden 20 g 2,6-Dichlor-3,5-difluorphenol vorgelegt und 8 g 98 %ige Salpetersäure zugetropft. Anschließend wird für 2 Stunden bei Raumtemperatur nachgerührt, in 150 ml Dichlormethan aufgenommen und zweimal mit Wasser gewaschen. Nach Abdestillieren des Dichlormethans verbleiben 18 g Produkt. Nach GC-Analyse 94 %ig.

Beispiel A7

2-Chlor-3,5,6-trifluor-4-nitrophenol

Analog Beispiel A5 werden durch Nitrierung aus 28 g 2-Chlor-3,5,6-trifluorphenol 25 g 2-Chlor-3,5,6-trifluor-4-nitrophenol erhalten mit einer Reinheit von 93 % und einem Schmelzpunkt von 107 - 109°C.

Beispiel A8

2,6-Dichlor-3,5-difluor-4-amino-phenol

18 g 2,6-Dichlor-3,5-difluor-4-nitrophenol werden in 100 ml Methanol in Gegenwart von 1,5 g Raney-Nickel bei 25 - 45°C mit 30 - 50 bar Wasserstoff bis zum Ende der Wasserstoffaufnahme hydriert. Nach Filtration wird die Lösung unter vermindertem Druck vom Lösungsmittel befreit. Es verbleiben 13 g Aminophenol (GC-Reinheit 98,4 %); Fp. 151°C.

Beispiel A9

2-Chlor-3,5,6-trifluor-4-amino-phenol

Analog Beispiel A7 werden aus 25 g 2-Chlor-3,5,6-trifluor-4-nitro-phenol in 120 ml Methanol und 2 g Raney-Nickel durch Hydrierung 20 g Aminophenol (GC-Reinheit 97 %) erhalten.

Analog Beispiel A1 werden die folgenden Verbindungen der Formel (IVa) erhalten.

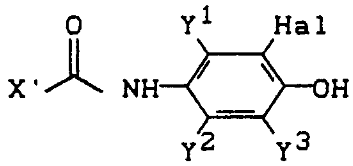

(IVa)

| Bsp.-Nr. | X' | $Y^1$ | $Y^2$ | $Y^3$ | Hal | phy. Daten |
|---|---|---|---|---|---|---|
| A11 | 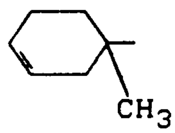 | Cl | H | H | Cl | |
| A12 | 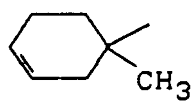 | Cl | H | Cl | H | |
| A13 | 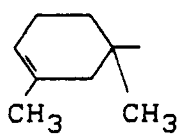 | Cl | H | H | Cl | |
| A14 | 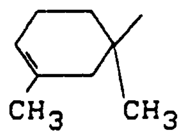 | Cl | H | Cl | H | |
| A15 | 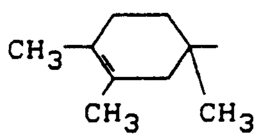 | Cl | H | Cl | H | |
| A16 | 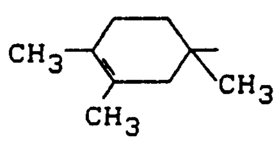 | Cl | H | H | Cl | |

Beispiel

Botrytis-Test (Bohne)/protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine ausgezeichnete Wirksamkeit zeigen in diesem Test z.B. die Verbindungen gemäß folgender Herstellungsbeispiele: 1, 2, 3, 4, 6, 7, 8, 9, 35 und 42.

## Patentansprüche

1. Cycloalkyl- bzw. Cycloalkenyl-carbonsäureanilide der allgemeinen Formel

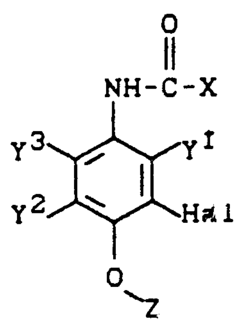

(I)

in welcher

| | |
|---|---|
| X | für gegebenenfalls Alkyl-substituiertes Cyclopentyl, Cyclohexyl oder Cycloheptyl oder gegebenenfalls Alkylsubstituiertes Cycloalkenyl steht, |
| Hal | für Halogen steht und |
| $Y^1$, $Y^2$ und $Y^3$ | unabhängig voneinander für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen und |
| Z | für die Gruppen $COOR^2$ oder $COR^1$ steht, wobei |
| $R^1$ und $R^2$ | gleich oder verschieden sind und für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl stehen. |

2. Verbindungen der Formel (I) gemäß Anspruch 1, worin

| | |
|---|---|
| X | für gegebenenfalls ein- bis vierfach, gleich oder verschieden durch geradkettiges oder verzweigtes Alkyl mit 1 - 4 Kohlenstoffatomen substituiertes Cyclopentyl, Cyclohexyl oder Cycloheptyl steht oder für Cycloalkenyl mit 5 - 7 Ringgliedern steht, wobei der Cycloalkylrest bzw. Cycloalke- |

nylrest ein- bis sechsfach , gleich oder verschieden durch geradkettiges oder verzweigtes Alkyl mit 1 - 4 Kohlenstoffatomen substituiert sein kann,

Hal für Fluor, Chlor oder Brom steht,

$Y^1$, $Y^2$ und $Y^3$ gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 -4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen,

Z für $COOR^2$ oder $COR^1$ steht, wobei

$R^1$ und $R^2$ gleich oder verschieden sind und für gegebenenfalls einfach bis neunfach durch Halogen substituiertes $C_1$-$C_8$-Alkyl, $C_2$-$C_8$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl stehen oder für unsubstituiertes oder einfach bis fünffach, gleich oder verschieden durch $C_1$-$C_4$-Halogenalkyl, Halogen, $C_1$-$C_4$-Alkyl, $C_1$-$C_4$-Alkoxy, Hydroxy, $C_1$-$C_6$-Alkylcarbonyloxy substituiertes $C_3$-$C_7$-Cycloalkyl stehen oder für unsubstituiertes oder im Phenylteil einfach bis fünffach, gleich oder verschieden durch $Y^{1'}$-$Y^{5'}$ substituiertes Phenyl-$C_1$-$C_4$-alkyl oder für unsubstituiertes oder im Phenylteil einfach bis fünffach, gleich oder verschieden durch $Y^{1'}$-$Y^{5'}$ substituiertes Phenoxyalkyl stehen,
wobei $Y^{1'}$-$Y^{5'}$ die Bedeutung von $Y^1$-$Y^3$, $NO_2$ und Cyano haben.

**3.** Verbindungen der Formel (I) gemäß Anspruch 1, worin

X für ein- oder zweifach, gleich oder verschieden durch geradkettiges oder verzweigtes Alkyl mit 1 - 4 Kohlenstoffatomen substituiertes Cyclopentyl, Cyclohexyl oder Cycloheptyl, für gegebenenfalls ein- oder zweifach, gleich oder verschieden durch geradkettiges oder verzweigtes Alkyl mit 1 - 4 Kohlenstoffatomen substituiertes Cyclopentenyl, Cyclohexenyl, Cycloheptenyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht,

Z für $COOR^2$ oder $COR^1$ steht, wobei
$R^1$ und $R^2$ gleich oder verschieden sind und für $C_1$-$C_6$-Alkyl, $C_2$-$C_6$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_2$-alkyl stehen oder für unsubstituiertes oder einfach bis dreifach, gleich oder verschieden durch Fluor, Chlor, Brom, $C_1$-$C_2$-Alkyl, $C_1$-$C_2$-Alkoxy, $C_1$-$C_6$-Alkylcarbonyloxy substituiertes $C_3$-$C_7$-Cycloalkyl stehen oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl-$C_1$-$C_2$-alkyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenoxy-$C_1$-$C_2$-alkyl stehen.

**4.** Verbindungen der Formel (I) gemäß Anspruch 1, worin

X für in 1- oder 1,3-Stellung durch Methyl oder Ethyl substituiertes Cyclopentyl, Cyclohexyl, Cycloheptyl, Cyclopentenyl, Cyclohexenyl, Cycloheptenyl steht, welche gegebenenfalls durch einen weiteren Alkylrest mit 1 - 3 Kohlenstoffatomen zusätzlich substituiert sind,

Hal für Fluor, Chlor oder Brom steht und

$Y^1$, $Y^2$ und $Y^3$ gleich oder verschieden sind und für Wasserstoff, Fluor, Chlor, Brom oder Trifluormethyl stehen,

Z für $COOR^2$ oder $COR^1$ steht, wobei
$R^1$ und $R^2$ gleich oder verschieden sind und für $C_1$-$C_4$-Alkyl, $C_2$-$C_5$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl stehen oder für einfach bis dreifach, gleich oder verschieden durch Methoxy, Fluor, Chlor, Brom, Methyl, $C_1$-$C_4$-Alkylcarbonyloxy substituiertes $C_3$-$C_7$-Cycloalkyl stehen oder für einfach bis dreifach, gleich oder verschieden durch Nitro, Cyano, Fluor, Chlor, Brom, Methyl, Methoxy substituiertes Phenyl oder für einfach bis dreifach, gleich oder verschieden durch Nitro, Cyano, Fluor, Chlor, Brom, Methyl, Methoxy substituiertes Phenylmethyl stehen.

**5.** Verfahren zur Herstellung von substituierten Cycloalkyl- bzw. Cycloalkenylcarbonsäureaniliden der Formel (I)

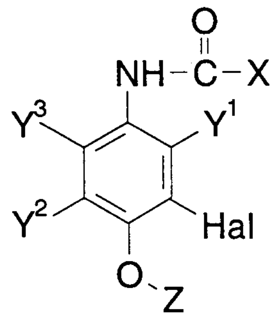

(I)

in welcher

X, $Y^1$, $Y^2$, $Y^3$, Hal und Z die in Anspruch 1 angegebenen Bedeutungen haben,

dadurch gekennzeichnet, daß man Aminophenole der Formel (II)

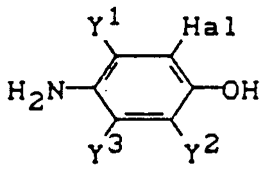

(II)

in welcher

Hal, $Y^1$, $Y^2$ und $Y^3$ die oben angegebenen Bedeutungen haben, in einem ersten Reaktionsschritt

mit Carbonsäure-Derivaten der Formel (III)

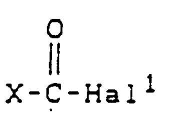

(III)

in welcher

X die oben angegebene Bedeutung hat und

$Hal^1$ für Halogen, oder eine bei Acylierungsreaktionen gebräuchliche Abgangsgruppe steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt diese erhaltenen Zwischenprodukte der For-

mel (IV)

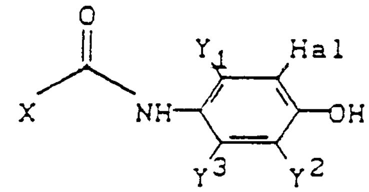

(IV)

in welcher

X, $Y^1$, $Y^2$, $Y^3$ und Hal die obengenannte Bedeutung haben,

mit Carbonyl-Derivaten der Formel (V)

$$Z\text{-}Hal^2 \quad (V)$$

in welcher

Z die obengenannte Bedeutung hat und

$Hal^2$ für Halogen oder eine bei Acylierungsreaktionen gebräuchliche Abgangsgruppe steht,

gegebenenfalls in Gegenwart eines Säureakzeptors und gegebenenfalls in Gegenwart eines Lösungs- oder Verdünnungsmittels umsetzt.

**6.** Schädlingsbekämpfungsmittel, gekennzeichnet durch einen Gehalt an mindestens einem Cycloalkyl- bzw. Cycloalkenyl-carbonsäureanilid der Formel (I) nach den Ansprüchen 1 bis 5.

**7.** Verwendung von Cycloalkyl- bzw. Cycloalkenylcarbonsäureanilid der Formel (I) nach den Ansprüchen 1 bis 5 zur Bekämpfung von Schädlingen.

**8.** Verfahren zur Bekämpfung von Schädlingen, dadurch gekennzeichnet, daß man Cycloalkyl- bzw. Cycloalkenylcarbonsäureanilid der Formel (I) nach den Ansprüchen 1 bis 5 auf Schädlinge und/oder ihren Lebensraum einwirken läßt.

**9.** Verfahren zur Herstellung von Schädlingsbekämpfungsmitteln, dadurch gekennzeichnet, daß man Cycloalkyl- bzw. Cycloalkenyl-carbonsäureanilid der Formel (I) nach den Ansprüchen 1 bis 5 mit Streckmitteln und/oder oberflächenaktiven Mitteln vermischt.

**10.** Acylaminophenole der Formel (IVa)

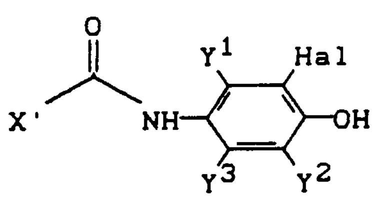

(IVa)

in welcher

X′ für einen gegebenenfalls Alkyl-substituierten Cycloalkenylrest, vorzugsweise einen $C_1$-$C_2$-Alkyl-substituierten Cyclopentenyl-, Cyclohexenyl- oder Cycloheptenylrest, steht, und

Hal für Halogen steht

$Y^1$, $Y^2$, $Y^3$ unabhängig voneinander für Wasserstoff, Halogen, gegebenenfalls Halogen-substituiertes Alkyl, gegebenenfalls Halogen-substiutiertes Alkoxy oder gegebenenfalls Halogen-substituiertes Alkylthio stehen.

## Claims

**1.** Cycloalkyl- or cycloalkenyl-carboxanilides of the general formula

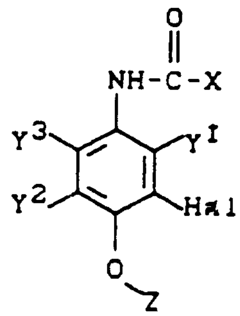

(I)

in which

X represents optionally alkyl-substituted cyclopentyl, cyclohexyl or cycloheptyl or optionally alkyl-substituted cycloalkenyl,

Hal represents halogen, and

$Y^1$, $Y^2$ and $Y^3$ independently of one another represent hydrogen, halogen, optionally halogen-substituted alkyl, optionally halogen-substituted alkoxy or optionally halogen-substituted alkylthio, and

Z represents the groups $COOR^2$ or $COR^1$, where

$R^1$ and $R^2$ are identical or different and represent optionally halogen-substituted alkyl, alkenyl, alkoxyalkyl, alkylthioalkyl, polyalkoxyalkyl and optionally substituted cycloalkyl, optionally substituted phenyl, optionally substituted phenylalkyl or substituted phenoxyalkyl.

**2.** Compounds of the formula (I) according to Claim 1, where

X represents cyclopentyl, cyclohexyl or cycloheptyl, each of which is optionally monosubstituted to tetrasubstituted by identical or different substituents from the series comprising straight-chain or branched alkyl radicals having 1 - 4 carbon atoms, or represents cycloalkenyl having 5 - 7 ring members, it being possible for the cycloalkyl radical, or cycloalkenyl radical, to be monosubstituted to hexasubstituted by identical or different substituents from the series comprising straight-chain or branched alkyl radicals having 1 - 4 carbon atoms,

Hal represents fluorine, chlorine or bromine,

$Y^1$, $Y^2$ and $Y^3$ are identical or different and represent hydrogen, fluorine, chlorine, bromine, straight-chain or branched alkyl having 1 - 4 carbon atoms, straight-chain or branched alkoxy or alkylthio each having 1 - 4 carbon atoms, or represent halogenoalkyl, halogenoalkoxy or halogenoalkylthio each having 1 - 4 carbon atoms in the straight-chain or branched alkyl moiety and having 1 - 5 identical or different halogen atoms,

Z represents $COOR^2$ or $COR^1$, where

$R^1$ and $R^2$ are identical or different and represent $C_1$-$C_8$-alkyl, $C_2$-$C_8$-alkenyl, $C_1$-$C_4$-alkoxy-$C_1$-$C_4$-alkyl, $C_1$-$C_4$-alkoxy-$C_1$-$C_4$-alkoxy-$C_1$-$C_4$-alkyl or $C_1$-$C_4$-alkylthio-$C_1$-$C_4$-alkyl, each of which is optionally monosubstituted to nonasubstituted by halogen, or represent $C_3$-$C_7$-cycloalkyl which is unsub-

stituted or monosubstituted to pentasubstituted by identical or different substituents from the series comprising $C_1$-$C_4$-halogenoalkyl, halogen, $C_1$-$C_4$-alkyl, $C_1$-$C_4$-alkoxy, hydroxyl and $C_1$-$C_6$-alkylcarbonyloxy, or represent phenyl-$C_1$-$C_4$-alkyl which is unsubstituted or monosubstituted to pentasubstituted in the phenyl moiety by identical or different substituents from the series comprising $Y^{1'}$ - $Y^{5'}$, or represent phenoxyalkyl which is unsubstituted or monosubstituted to pentasubstituted in the phenyl moiety by identical or different substituents from the series comprising $Y^{1'}$ - $Y^{5'}$,
where $Y^{1'}$ - $Y^{5'}$ have the meaning of $Y^1$ - $Y^3$, $NO_2$ and cyano.

**3.** Compounds of the formula (I) according to Claim 1, where

X represents cyclopentyl, cyclohexyl or cycloheptyl, each of which is monosubstituted or disubstituted by identical or different substituents from the series comprising straight-chain or branched alkyl radicals having 1 - 4 carbon atoms, or represents cyclopentenyl, cyclohexenyl or cycloheptenyl, each of which is optionally monosubstituted or disubstituted by identical or different substituents from the series comprising straight-chain or branched alkyl radicals having 1 - 4 carbon atoms,

$Y^1$, $Y^2$ and $Y^3$ identical or different and represent hydrogen, fluorine, chlorine, bromine, methyl or trifluoromethyl, and

Hal represents fluorine, chlorine or bromine,

Z represents $COOR^2$ or $COR^1$, where
$R^1$ and $R^2$ are identical or different and represent $C_1$-$C_6$-alkyl, $C_2$-$C_6$-alkenyl, $C_1$-$C_4$-alkoxy-$C_1$-$C_4$-alkyl, $C_1$-$C_4$-alkoxy-$C_1$-$C_4$-alkoxy-$C_1$-$C_4$-alkyl or $C_1$-$C_4$-alkylthio-$C_1$-$C_2$-alkyl, or represent $C_3$-$C_7$-cycloalkyl which is unsubstituted or monosubstituted to trisubstituted by identical or different substituents from the series comprising fluorine, chlorine, bromine, $C_1$-$C_2$-alkyl, $C_1$-$C_2$-alkoxy or $C_1$-$C_6$-alkylcarbonyloxy, or represent phenyl which is unsubstituted or monosubstituted to trisubstituted by identical or different substituents from the series comprising nitro, cyano, fluorine, chlorine, bromine, methyl, trifluoromethyl, trifluoromethylthio, methoxy and trifluoromethoxy, or represent phenyl-$C_1$-$C_2$-alkyl which is unsubstituted or monosubstituted to trisubstituted by identical or different substituents from the series comprising nitro, cyano, fluorine, chlorine, bromine, methyl, trifluoromethyl, trifluoromethylthio, methoxy and trifluoromethoxy, or represent phenoxy-$C_1$-$C_2$-alkyl which is unsubstituted or monosubstituted to trisubstituted by identical or different substituents from the series comprising nitro, cyano, fluorine, chlorine, bromine, methyl, trifluoromethyl, trifluoromethylthio, methoxy and trifluoromethoxy.

**4.** Compounds of the formula (I) according to Claim 1, where

X represents cyclopentyl, cyclohexyl, cycloheptyl, cyclopentenyl, cyclohexenyl or cycloheptenyl, each of which is substituted in the 1- or 1,3-position by methyl or ethyl and each of which is optionally additionally substituted by a further alkyl radical having 1 - 3 carbon atoms,

Hal represents fluorine, chlorine or bromine, and

$Y^1$, $Y^2$ and $Y^3$ are identical or different and represent hydrogen, fluorine, chlorine, bromine or trifluoromethyl,

Z represents $COOR^2$ or $COR^1$, where
$R^1$ and $R^2$ are identical or different and represent $C_1$-$C_4$-alkyl, $C_2$-$C_5$-alkenyl, $C_1$-$C_4$-alkoxy-$C_1$-$C_4$-alkyl or $C_1$-$C_4$-alkoxy-$C_1$-$C_4$-alkoxy-$C_1$-$C_4$-alkyl, or represent $C_3$-$C_7$-cycloalkyl which is monosubstituted to trisubstituted by identical or different substituents from the series comprising methoxy, fluorine, chlorine, bromine, methyl and $C_1$-$C_4$-alkylcarbonyloxy, or represent phenyl which is monosubstituted to trisubstituted by identical or different substituents from the series comprising nitro, cyano, fluorine, chlorine, bromine, methyl and methoxy, or represent phenylmethyl which is monosubstituted to trisubstituted by identical or different substituents from the series comprising nitro, cyano, fluorine, chlorine, bromine, methyl and methoxy.

**5.** Process for the preparation of substituted cycloalkyl- or cycloalkenylcarboxanilides of the formula (I)

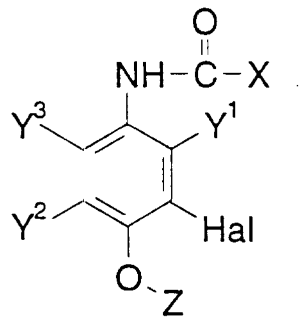

(I)

in which

X, $Y^1$, $Y^2$, $Y^3$, Hal and Z have the meanings given in Claim 1,

characterized in that aminophenols of the formula (II)

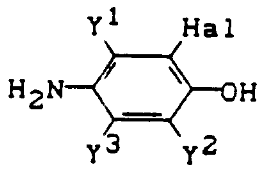

(II)

in which

Hal, $Y^1$, $Y^2$ and $Y^3$ have the abovementioned meanings, are reacted, in a first reaction step, with carboxylic acid derivatives of the formula (III)

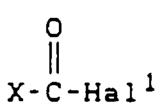

(III)

in which

X has the abovementioned meaning and

$Hal^1$ represents halogen, or represents a leaving group customary in acylation reactions,

if appropriate in the presence of an acid acceptor and if appropriate in the presence of a solvent or diluent, and these resulting intermediates of the formula (IV)

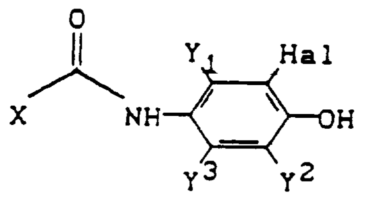

(IV)

in which

X, $Y^1$, $Y^2$, $Y^3$ and Hal have the abovementioned meaning, are then reacted, in a second reaction step, with carbonyl derivatives of the formula (V)

$$Z\text{-}Hal^2 \qquad (V)$$

in which

Z has the abovementioned meaning and

$Hal^2$ represents halogen or a leaving group customary in acylation reactions,

if appropriate in the presence of an acid acceptor and if appropriate in the presence of a solvent or diluent.

**6.** Pesticides, characterized in that they contain at least one cycloalkyl- or cycloalkenyl-carboxanilide of the formula (I) according to Claims 1 to 5.

**7.** Use of cycloalkyl- or cycloalkenyl-carboxanilide of the formula (I) according to Claims 1 to 5, for combating pests.

**8.** Method of combating pests, characterized in that cycloalkyl- or cycloalkenyl-carboxanilide of the formula (I) according to Claims 1 to 5 is allowed to act on pests and/or their habitat.

**9.** Process for the preparation of pesticides, characterized in that cycloalkyl- or cycloalkenylcarboxanilide of the formula (I) according to Claims 1 to 5 is mixed with extenders and/or surface-active agents.

**10.** Acylaminophenols of the formula (IVa)

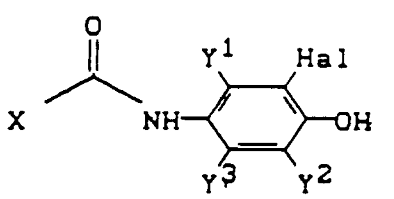

(IVa)

in which

X' represents an optionally alkyl-substituted cycloalkenyl radical, preferably a $C_1$-$C_2$-alkyl-substituted cyclopentenyl radical, cyclohexenyl radical or cycloheptenyl radical, and

Hal represents halogen, and

$Y^1$, $Y^2$ and $Y^3$ independently of one another represent hydrogen, halogen, optionally halogen-substituted alkyl, optionally halogen-substituted alkoxy or optionally halogen-substituted alkylthio.

## Revendications

1. Anilides d'acides cycloalkyl- et cycloalcénylcarboxyliques de formule générale

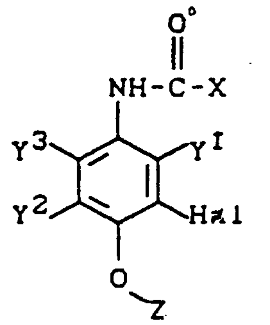

(I)

dans laquelle

| | |
|---|---|
| X | désigne un groupe cyclopentyle, cyclohexyle ou cycloheptyle éventuellement substitué par un radical alkyle ou un groupe cycloalcényle éventuellement substitué par un radical alkyle, |
| Hal | représente un halogène et |
| $Y^1$, $Y^2$ et $Y^3$ | représentent, indépendamment les uns des autres, l'hydrogène, un halogène, un groupe alkyle éventuellement substitué par un halogène, un groupe alkoxy éventuellement substitué par un halogène ou un groupe alkylthio éventuellement substitué par un halogène et |
| Z | représente les groupes $COOR^2$ ou $COR^1$. dans lesquels |
| $R^1$ et $R^2$ | sont identiques ou différents et représentent un groupe alkyle, alcényle, alkoxyalkyle, alkylthioalkyle, polyalkoxyalkyle éventuellement substitué par un halogène et un groupe cycloalkyle éventuellement substitué, un groupe phényle éventuellement substitué, un groupe phénylalkyle éventuellement substitué ou un groupe phénoxyalkyle substitué. |

2. Composés de formule (I) suivant la revendication 1,
dans lesquels

| | |
|---|---|
| X | représente un groupe cyclopentyle, cyclohexyle ou cycloheptyle éventuellement substitué 1 à 4 fois identiques ou différentes par un radical alkyle linéaire ou ramifié ayant 1 à 4 atomes de carbone, ou un groupe cycloalcényle de 5 à 7 chaînons, le reste cycloalkyle ou le reste cycloalcényle pouvant être substitué 1 à 6 fois identiques ou différentes par un radical alkyle linéaire ou ramifié ayant 1 à 4 atomes de carbone, |
| Hal | représente le fluor, le chlore ou le brome, |
| $Y^1$, $Y^2$ et $Y^3$ | sont identiques ou différents et représentent de l'hydrogène, du fluor, du chlore, du brome, un groupe alkyle linéaire ou ramifié ayant 1 à 4 atomes de carbone, un groupe alkoxy ou alkylthio linéaire ou ramifié ayant chacun 1 à 4 atomes de carbone, un groupe halogénalkyle, halogénalkoxy ou halogénalkylthio ayant chacun 1 à 4 atomes de carbone dans la partie alkyle linéaire ou ramifié et 1 à 5 atomes d'halogènes identiques ou différents, |
| Z | est un groupe $COOR^2$ ou $COR^1$, où |
| $R^1$ et $R^2$ | sont identiques ou différents et représentent un groupe alkyle en $C_1$ à $C_8$, alcényle en $C_2$ à $C_8$, (alkoxy en $C_1$ à $C_4$)-(alkyle en $C_1$ à $C_4$), alkoxy en $C_1$ à $C_4$)-(alkoxy en $C_1$ à $C_4$)-(alkyle en $C_1$ à $C_4$), (alkylthio en $C_1$ à $C_4$)-(alkyle en $C_1$ à $C_4$ substitué le cas échéant 1 à 9 fois par un halogène, ou un groupe cycloalkyle en $C_3$ à $C_7$, non substitué ou substitué 1 à 5 fois identiques ou différentes par un radical halogénalkyle en $C_1$ à $C_4$, halogéno, alkyle en $C_1$ à $C_4$, alkoxy en $C_1$ à $C_4$, hydroxy, (alkyle en $C_1$ à $C_6$)carbonyloxy, ou un groupe phényl-(alkyle en $C_1$ à $C_4$) non substitué ou substitué 1 à 5 fois identiques ou différentes dans la partie phényle par les radicaux $Y^{1}$'-$Y^{5}$' ou un groupe phénoxyalkyle non substitué ou substitué 1 à 5 fois identiques ou différentes dans la partie phényle par des radicaux $Y^{1}$'-$Y^{5}$' ayant la définition des groupes $Y^1$-$Y^3$, $NO_2$ et cyano. |

3. Composés de formule (I) suivant la revendication 1,
dans lesquels

X représente un groupe cyclopentyle, cyclohexyle ou cycloheptyle substitué 1 ou 2 fois identiques ou différentes par un radical alkyle en $C_1$ à $C_4$ linéaire ou ramifié, un groupe cyclopentényle, cyclohexényle ou cycloheptényle éventuellement substitué 1 ou 2 fois identiques ou différentes par un radical alkyle linéaire ou ramifié ayant 1 à 4 atomes de carbone,

$Y^1$, $Y^2$ et $Y^3$ sont identiques ou différents et représentent de l'hydrogène, du fluor, du chlore, du brome, un groupe méthyle ou trifluorométhyle et

Hal représente du fluor, du chlore ou du brome,

Z est un groupe $COOR^2$ ou $COR^1$, dans lequel $R^1$ et $R^2$ sont identiques ou différents et représentent un groupe alkyle en $C_1$ à $C_6$, alcényle en $C_2$ à $C_6$, (alkoxy en $C_1$ à $C_4$)-(alkyle en $C_1$ à $C_4$), (alkoxy en $C_1$ à $C_4$)-(alkoxy en $C_1$ à $C_4$)-(alkyle en $C_1$ à $C_4$), (alkylthio en $C_1$ à $C_4$)-(alkyle ou $C_1$ ou $C_2$) ou un groupe cycloalkyle en $C_3$ à $C_7$ non substitué ou substitué 1 à 3 fois, identiques ou différentes, par du fluor, du chlore, du brome, un radical alkyle en $C_1$ ou $C_2$, alkoxy en $C_1$ ou $C_2$, (alkyle en $C_1$ à $C_6$)carbonyloxy, ou un groupe phényle non substitué ou substitué 1 à 3 fois identiques ou différentes par un radical nitro, cyano, fluoro, chloro, bromo, méthyle, trifluorométhyle, trifluorométhylthio, méthoxy, trifluorométhoxy, ou un groupe phényl-(alkyle en $C_1$ ou $C_2$) non substitué ou substitué 1 à 3 fois identiques ou différentes par un radical nitro, cyano, fluoro, chloro, bromo, méthyle, trifluorométhyle, trifluorométhylthio, méthoxy, trifluorométhoxy, ou un groupe phénoxy-(alkyle en $C_1$ ou $C_2$) non substitué ou substitué 1 à 3 fois identiques ou différentes par un radical nitro, cyano, fluoro, chloro, bromo, méthyle, trifluorométhyle, trifluorométhylthio, méthoxy, trifluorométhoxy.

**4.** Composés de formule (I) suivant la revendication 1, dans lesquels

X représente un groupe cyclopentyle, cyclohexyle, cycloheptyle, cyclopentényle, cyclohexényle, cycloheptényle, substitué en position 1 ou en positions 1, 3 par un reste méthyle ou éthyle, ces groupes étant en outre substitués le cas échéant par un autre reste alkyle ayant 1 à 3 atomes de carbone,

Hal représente le fluor, le chlore ou le brome et

$Y^1$, $Y^2$ et $Y^3$ sont identiques ou différents et représentent de l'hydrogène, du fluor, du chlore, du brome ou un groupe trifluorométhyle,

Z est un groupe $COOR^2$ ou $COR^1$ dans lequel $R^1$ et $R^2$ sont identiques ou différents et représentent un groupe alkyle en $C_1$ à $C_4$, alcényle en $C_2$ à $C_5$, (alkoxy en $C_1$ à $C_4$)-(alkyle en $C_1$ à $C_4$), (alkoxy en $C_1$ à $C_4$)-(alkoxy en $C_1$ à $C_4$)-(alkyle en $C_1$ à $C_4$) ou un groupe cycloalkyle en $C_3$ à $C_7$ subtitué 1 à 3 fois identiques ou différentes par un radical méthoxy, fluoro, chloro, bromo, méthyle, (alkyle en $C_1$ à $C_4$)carbonyloxy ou un groupe phényle substitué 1 à 3 fois identiques ou différentes par un radical nitro, cyano, fluoro, chloro, bromo, méthyle, méthoxy, ou un groupe phénylméthyle substitué 1 à 3 fois identiques ou différentes par un radical nitro, cyano, fluoro, chloro, bromo, méthyle, méthoxy.

**5.** Procédé de production d'anilides d'acides cycloalkyl- ou cycloalcénylcarboxyliques substitués de formule (I)

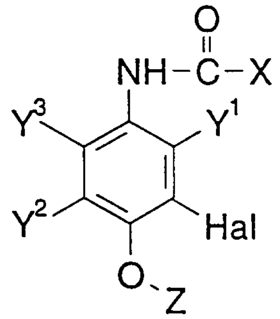

(I)

dans laquelle

X, $Y^1$, $Y^2$, $Y^3$, Hal et Z ont les définitions indiquées dans la revendication 1,

caractérisé en ce qu'on fait réagir des aminophénols de formule (II)

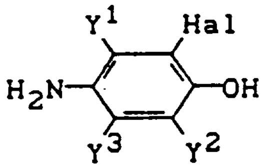

(II)

dans laquelle

Hal, $Y^1$, $Y^2$ et $Y^3$ ont les définitions indiquées ci-dessus, dans une première étape réactionnelle,

avec des dérivés d'acides carboxyliques de formule (III)

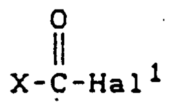

(III)

dans laquelle

X a la définition indiquée ci-dessus et
$Hal^1$ représente un halogène ou un groupe partant classique dans des réactions d'acylation,

le cas échéant en présence d'un accepteur d'acide et en la présence éventuelle d'un solvant et d'un diluant, puis on fait réagir dans une seconde étape de réaction ces produits intermédiaires obtenus de formule (IV)

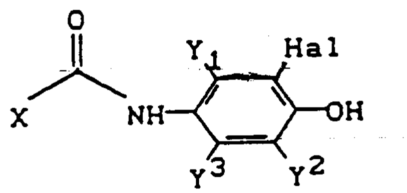

(IV)

dans laquelle

X, $Y^1$, $Y^2$, $Y^3$ et Hal ont la définition indiquée ci-dessus, avec des dérivés carbonyliques de formule (V)

$$Z\text{-}Hal^2 \qquad (V)$$

dans laquelle

Z a la définition indiquée ci-dessus et
$Hal^2$ représente un halogène ou un groupe partant classique dans des réactions d'acylation,

le cas échéant en présence d'un accepteur d'acide et en la présence éventuelle d'un solvant ou d'un diluant.

6. Compositions pesticides, caractérisées par une teneur en au moins un anilide d'acide cycloalkyl- ou cycloalcénylcarboxylique de formule (I) suivant les revendications 1 à 5.

7. Utilisation d'un anilide d'acide cycloalkyl-ou cycloalcénylcarboxylique de formule (I) suivant les revendications 1 à 5 pour combattre des parasites.

**8.** Procédé pour combattre des parasites, caractérisé en ce qu'on fait agir un anilide d'acide cycloalkyl- ou cycloalcénylcarboxylique de formule (I) suivant les revendications 1 à 5 sur des parasites et/ou sur leur milieu.

**9.** Procédé de préparation de compositions pesticides, caractérisé en ce qu'on mélange un anilide d'acide cycloalkyl- ou cycloalcénylcarboxylique de formule (I) suivant les revendications 1 à 5 avec des diluants et/ou des agents tensio-actifs.

**10.** Acylaminophénols de formule (IVa)

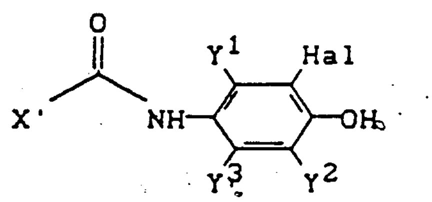

(IVa)

dans laquelle

X représente un reste cycloalcényle portant éventuellement un substituant alkyle, un reste cyclopentényle, cyclohexényle ou cycloheptényle portant de préférence un substituant alkyle en $C_1$ ou $C_2$, et

Hal représente un halogène,

$Y^1$, $Y^2$, $Y^3$ représentent indépendamment les uns des autres de l'hydrogène, un halogène, un groupe alkyle éventuellement substitué par un halogène, un groupe alkoxy éventuellement substitué par un halogène ou un groupe alkylthio éventuellement substitué par un halogène.